# EUROPEAN PATENT SPECIFICATION

(11) **EP 2 825 545 B1**
(45) Date of publication and mention of the grant of the patent: **02.05.2018**
(21) Application number: 13720074.7
(22) Date of filing: 13.03.2013
(51) Int. Cl.: C07H 1/08, C13K 13/00

(54) **PROCESS FOR THE RECOVERY OF L-FUCOSE FROM EXOPOLYSACCHARIDES**
VERFAHREN ZUR RÜCKGEWINNUNG VON L-FUKOSE AUS EXOPOLYSACCHARIDEN
PROCÉDÉ DE RÉCUPÉRATION DE L-FUCOSE À PARTIR D'EXOPOLYSACCHARIDES

(30) Priority: 13.03.2012 IT FI20120052
(43) Date of publication of application: 21.01.2015
(73) Proprietor: INALCO S.R.L., 20139 Milano (IT)
(72) Inventor: VAGNOLI, Luana, I-52040 Quarata - Arezzo (IT); GIACOMELLI, Silvia, I-50019 Sesto Fiorentino (IT); BIAGIOLINI, Silvia, I-52100 Arezzo (IT); CIPOLLETTI, Giovanni, I-20143 Milano (IT); GORI, Alessandro, I-50139 Firenze (IT)
(74) Representative: Valenza, Silvia
(86) International application number: PCT/IB2013/051994
(87) International publication number: WO 2013/136280

(56) References cited:
- WO-A1-2008/127134
- WO-A1-2012/034996
- US-A1- 2011 159 288
- VAN DEN BULK R W ET AL: "CHARACTERIZATION OF THE EXTRACELLULAR POLYSACCHARIDE PRODUCED BY CLAVIBACTER-MICHIGANENSIS-SSP-MICHIGANENSI S", PHYTOPATHOLOGY, AMERICAN PHYTOPATHOLOGICAL SOCIETY, US, vol. 81, no. 6, 1 January 1991 (1991-01-01), pages 619-623, XP002638647, ISSN: 0031-949X, DOI: 10.1094/PHYTO-81-619
- MCCONVILLE M J ET AL: "Subcellular location and composition of the wall and secreted extracellular sulphated polysaccharides/proteoglycans of the diatom Stauroneis amphioxys Gregory", PROTOPLASMA, vol. 206, no. 1-3, 1999, pages 188-200, XP009164269, ISSN: 0033-183X

## Description

### FIELD OF THE INVENTION

The present invention relates to the sugar production field, in particular the preparation of L-fucose; more in particular, the present invention relates to the isolation, on an industrial scale, of L-Fucose (or 6-Deoxygalactose) starting from exopolysaccharides obtained by fermentative process.

### BACKGROUND OF THE INVENTION

Interest in L-Fucose, particularly on the part of the nutraceutical industry, is currently very high, due to fucosyl-derivatives compound properties such as anti-allergic, moisturising, emulsifying, stabilizing properties. L-Fucose represents the starting material for these fucosyl-derivative compounds. Of particular importance is the use of L-Fucose as precursor of analogous semi-synthetics of the fucosylated oligosaccharides contained in human milk, the so-called Human milk oligosaccharides (HMO).

L-Fucose currently present on the market is obtained from natural sources, in particular by acid hydrolysis of sulphated polysaccharides such as Fucoidans extracted from algae such as *Laminaria.* However, direct extraction from the algae is costly and also subject to seasonal variations of product volumes and quality. Another source of L-fucose is represented by the bark of standard trees such as willow, birch and beech. In this case L-fucose is again extracted by hydrolysis with low yields and high costs.

Chemical syntheses for preparing L-Fucose are known, which often pass from the configuration inversion of a highly available sugar, always providing for a number of steps and the use of very costly reagents.

Processes for preparing L-Fucose by alternative routes provide for the use of enzymes obtained from the engineering of suitable microorganisms.

The formation processes of L-Fucose by fermentation route are also known. Some extracellular microbial polysaccharides (EPS), better known for their properties as thickening, gelling or emulsifying agents, constitute an attractive source of L-fucose. There are several known microbial strains, especially of the Entrobacter genus, which produce exopolysaccharides containing L-fucose.

Just some of the many examples of EPS fermentative production containing L-fucose are provided below:
P. T. Vanhooren et al Med. Fac.Landbouww. Univ.Gent, 62/4a,1997 describe fermentation with *Clavibacter michiganensis* subsp. *Michiganensis.*
M.E. Pintado et al- Journal of Food Science - Vol. 64, N° 2, 1999 describe fermentation with *Rhanella aquatilis.*
Cristiana A.V. Torres et al Journal of Biotechnology - 156 (2011) 261-267 describe fermentation with *Enterobacter*
Filomena Freitas et al Bioresource Technology - 100 (2009) 859-865 describe fermentation with the *Pseudomonas* strain
FR 2840920 describes fermentation with a new microorganism of the Enterobacteriaceae family NO. I-2744, deposited in the Collection Nationale des Cultures de Microorganismes, called BEC 1645.
US 4806636 describes fermentation with *Enterobacter sakazakii*
US 5876982 describes fermentation with *Klebsiella pneumoniae* subsp. *Pneumonite.*
WO2008/127134 describes a galactose-rich polymer and process for preparing it. According to the process described therein "at the end of the fermentation the galactose rich polymer may be recovered directly from the culture broth, simply by drying" or alternatively may be precipitated from the culture broth or alternatively centrifugation followed by precipitation.

However, despite all of these methods known in the prior art, the obtaining of L-fucose, excluding the methods of extraction from natural sources, is inapplicable at industrial level to date.

In particular, as regards the obtaining of L-fucose from EPS, it is known that exopolysaccharides containing L-Fucose have molecular weight distributed between 10⁴ and 10⁸ and give rise to fermentation broths characterized by a high viscosity (greater than 500 ÷ 1000cps). The subsequent processing is described solely on laboratory scale and provides for the dilution of the mixture 1-2 times and subsequent centrifugation at >10000 rpm, to remove the cells, and the polysaccharide is subsequently precipitated by the addition of a solvent. On an industrial scale, the processing of these broths would require the processing of enormous volumes of work and the use of equipment such as centrifuges with a very high number of revolutions that have a significant economic impact on process costs; considerable volumes of solvents that would have a not negligible environmental impact would also be required.

The object of the present invention is to provide a process for isolating L-Fucose from EPS that is feasible on an industrial scale.

### SUMMARY OF THE INVENTION

The present invention resolves the above-mentioned problems by a process for isolating L-fucose from a polysaccharide comprising L-fucose, said polysaccharide contained in a harvest fermentation broth of a microorganism, said process characterized in that the harvest fermentation broth is subjected, as a first purification step, to ultrafiltration for isolating the polysaccharide, together with the cells, in the retentate, and to quantitatively removing in the permeate salts and residues of the various carbon sources which may have been used for the fermentation together with the by-products resulting from the fermentation; excluding the polysaccharide contained in the harvest fermentation broth of microorganism DMS22227.

Fermentation broth means a harvest fermentation broth, i.e. the mixture obtained at the end of the fermentation of a certain microorganism, said mixture containing the polysaccharide of interest (i.e. to be collected) in amounts sufficient to permit the isolation thereof.

The present invention provides a simple process which, through the above-mentioned ultrafiltration, allows processing with low volumes in the absence of solvents other than water and with widely diffused industrial machinery that have low maintenance costs, are easy to use and offer great flexibility of use. The process is therefore practical on an industrial scale at contained costs, with low environmental impact and offering an L-fucose with high degrees of purity.

The present invention also relates to a process for isolating a polysaccharide comprising L-fucose and contained in a fermentation broth of a microorganism, said process characterized in that the harvest fermentation broth is subjected, as a first purification step, to ultrafiltration to isolate the polysaccharide, together with the cells, in the retentate, and to quantitatively remove in the permeate the salts and residues of the various carbon sources that may have been used for the fermentation together with the by-products resulting from the fermentation; excluding the polysaccharide contained in the fermentation broth of microorganism DMS22227.

### DETAILED DESCRIPTION OF THE INVENTION

The purification step is a key point of the L-Fucose recovery process: the polysaccharide is recovered by ultrafiltration in that it quantitatively remains in the retentate together with only the cells of the microorganism while all the culture medium residues, the fermentation by-products and the salts are eliminated in the permeate in an almost quantitative manner.

This step excellently resolves the problem of the purification of the Fucose with a view to obtaining a high-purity crystal; it indeed allows separation of the sugars that make up the polysaccharide from all those by-products having a low molecular weight that cannot be eliminated in the subsequent steps and which, being present in considerable amounts, constitute a great problem for the direct crystallization of the product of the product.

For a preferred aspect, the process of the invention relates to polysaccharides contained in fermentation broths of microorganisms belonging to the *Enterobacteror Clavibacter* genus; preferably the above-mentioned bacteria *Clavibacter michiganensis* subsp. *Michiganensis, Rhanella aquatilis Pseudomonas* strain, Enterobacteriaceae No. 1-2744 deposited in the Collection Nationale des Cultures de Microorganismes called BEC 1645, *Enterobacter sakazakii, Klebsiella pneumoniae* subsp. *Pneumonite, Enterobacter A47-DSM23139.*

Ultrafiltration of the fermentation broth can be performed either on the broth as is or after having subjected the broth to fluidisation treatments that can also be used in combination with each other. Dilution greater than 0.5 times the volume of the broth is to be understood as excluded from these treatments.

These treatments can be thermal (heating at temperatures in excess of 50°C), mechanical (vigorous agitation at more than 180 rpm), physicochemical (acidification to pH of less than or equal to 3.5).

In a particularly preferred and advantageous manner, the fermentation broth, containing the polysaccharide comprising L-Fucose is acidified to a pH of between 3.5 and 1.5 before being ultrafiltered. In particular, it is convenient to combine heating at temperatures between 50 and 70 °C when acidification leads to a pH of between 3.5 and 2.0. When the pH is less than 2.0 it is not recommended to also combine the heating the mixture.

For the above-mentioned acidification treatment, strong acids can be used, even at maximum concentrations possible such as for example Sulfuric Acid, Hydrochloric Acid, Phosphoric Acid, Trifluoroacetic Acid. Sulfuric acid is preferred. The afore-mentioned acidification treatment produces a lowering of the viscosity at values less than 100 cps while maintaining the volumes of the mixture practically unaltered. As a result of the ultrafiltration, the retentate presents volumes equal to about one fourth with respect to the volumes of the fermentation broth.

The membranes used can be of various types and cut-offs.

Depending on the type of machine used and on the operating pressures, efficient spiral membranes with molecular cut-off of 5000 or 20000 Daltons or 0.05 µm ceramic membranes can be efficient.

Identification of the most suitable membrane for isolating the polysaccharide is a function of the molecular weight of the polysaccharide and also of its quaternary structure, thus various solutions for proceeding with isolation of the molecule can be identified.

On the retentate containing the polysaccharide, comprising L-Fucose, the purification process of L-Fucose continues according to known methods (see for example EP 102535, H. Voelskow and M. Schlingmann).

A full hydrolysis is carried out by means of a strong acid (for example, Sulfuric Acid, Hydrochloric Acid, Phosphoric Acid, Trifluoroacetic Acid, etc.). Sulfuric acid is preferred.

Once hydrolysis is complete, the mixture is neutralized by the addition of a base (Sodium Hydroxide, Calcium Hydroxide, etc.). Calcium hydroxide is preferred.

In one preferred aspect of the invention Sulfuric Acid and Calcium Hydroxide are used in combination: there is thus precipitation of the CaSO₄ that is removed by filtration thus eliminating large part of the salts present in the reaction mixture. The elimination of Glucose and Galactose from the mixture is obtained by techniques known to those skilled in the art, for example by chromatography or, preferably, through the action of a microorganism that exploits these carbohydrates as a source of carbon in order to grow, while leaving the fucose intact. Preferably the inoculation of the solution is carried out with *Saccharomyces cerevisiae.*

Fermentation is carried out until the complete disappearance of Glucose and Galactose. The yeast cells are removed from the L-Fucose solution by ultrafiltration, and the ultrafiltered solution is deionised by the passage on ionexchange resins, strong cationic and weak anionic arranged in series.

The deionised solution is concentrated to a syrup and L-Fucose (the HPLC purity of which in the solution in this phase is greater than 75%), is crystallized by the addition of solvent, for example alcohols such as Methanol, Ethanol, n-Propanol, Isopropanol or 2-Butanone, or, at the expense of the crystallization yields, of water directly.

At the end of the process it is possible to obtain L-Fucose, having HPLC titre > 99.5 % and HPLC area purity > 99.9 %, with yields greater than 80%

In the event of a desire to isolate the polysaccharide without obtaining L-Fucose therefrom, following ultrafiltration, the above-mentioned process rather than proceeding with polysaccharide hydrolysis, can instead continue by means of an additional filtration, with any system known to those skilled in the art, which is capable of holding the cells and allowing the polysaccharide to pass; it will thus, for example, be possible to microfilter, vacuum filter on a dicalite panel or centrifuge.

## Claims

1. A process for isolating L-fucose from a polysaccharide, said polysaccharide comprising L-Fucose, said polysaccharide contained in a harvest fermentation broth of a microorganism, said process **characterized in that** the harvest fermentation broth is subjected, as a first purification treatment, to ultrafiltration for isolating the polysaccharide along with the cells in the retentate, and quantitatively removing in the permeate salts and residues of the various carbon sources, which may have been used for the fermentation along with the by-products resulting from the fermentation;
wherein the ultrafiltration is carried out on the harvest fermentation broth as it is obtained at the end of the harvest fermentation or after having subjected the broth as it is obtained at the end of the fermentation to fluidization treatments;
excluding the polysaccharide contained in the harvest fermentation broth of microorganism DMS22227.

2. A process according to claim 1, wherein the microorganism is belonging to the *Enterobacter* or *Clavibacter* genus.

3. A process according to claim 2, wherein the fluidization treatments are selected from thermal, mechanical and chemical processes or combination thereof.

4. A process according to 3, wherein the harvest fermentation broth is acidified to a pH from 3.5 to 1.5 prior to being ultrafiltered.

5. A process according to claim 4, wherein the acidification to a pH from 3.5 to 2.0 is combined with heating at temperatures from 50 to 70 °C.

6. A process according to claim 4 or 5, wherein a strong acid is also used at its maximum possible concentration, for example selected from Sulfuric Acid, Hydrochloric Acid, Phosphoric Acid, Trifluoroacetic Acid.

7. A process according to any one of claims 1-6, wherein spiral membranes are used, with molecular weight cut off from 5000 to 300000 Daltons.

8. A process according to any one of claims 1-7, wherein the ultrafiltration is followed by complete hydrolysis of the polysaccharide by means of a treatment with a strong acid.

9. A process according to claim 8, wherein the hydrolysis is followed by neutralization with a base.

10. A process according to claim 9, wherein the neutralized mixture is subjected to a treatment for removing glucose, galactose or other sugars which form the polysaccharide.

11. A process according to claim 10, wherein after optional filtration or ultrafiltration, the resulting mixture is deionized by passing on ionic-exchange resins, and finally the L-fucose is crystallized by adding a solvent selected from Methanol, Ethanol, n-Propanol, Isopropanol or 2-Butanone, or directly in water.

12. A process for isolating an extracellular polysaccharide comprising L-Fucose and contained in a harvest fermentation broth of a microorganism, said process **characterized in that** the harvest fermentation broth is subjected, as a first purification step, to ultrafiltration for isolating the polysaccharide along with the cells in the retentate, and quantitatively removing in the permeate salts and residues of the various carbon sources, which may have been used for the fermentation along with the by-products resulting from the fermentation; wherein the ultrafiltration is carried out on the harvest fermentation broth as it is obtained at the end of the harvest fermentation or after having subjected the broth as it is obtained at the end of the fermentation to fluidization treatments;
excluding the polysaccharide contained in the harvest fermentation broth of microorganism DMS22227.

## Patentansprüche

1. Verfahren zum Abtrennen von L-Fukose von einem Polysaccharid, das L-Fukose enthält, wobei das Polysaccharid in einer Ernte-Fermentationslösung eines Mikroorganismus enthalten ist und das Verfahren **dadurch gekennzeichnet ist, dass** die Ernte-Fermentationslösung für eine erste Reinigung einer Ultrafiltration unterzogen wird, um im Retentat das Polysaccharid zusammen mit den Zellen abzutrennen und um im Permeat quantitativ Salze und Rückstände der verschiedenen Kohlenstoffquellen, die bei der Fermentation benutzt wurden, sowie die Nebenprodukte der Fermentation zu entfernen;
wobei die Ultrafiltration durchgeführt wird mit der Ernte-Fermentationslösung, wie sie am Ende der Ernte-Fermentation erhalten wird, oder nachdem die Lösung, wie sie am Ende der Fermentation erhalten wird, Fluidisierungs-Behandlungen unterzogen wurde,
wobei das Polysaccharid in der Ernte-Fermentationslösung des Mikroorganismus DMS22227 ausgenommen ist.

2. Verfahren gemäß Anspruch 1, wobei der Mikroorganismus zur Gattung *Enterobacter* oder *Clavibacter* gehört.

3. Verfahren gemäß Anspruch 2, wobei die Fluidisierungs-Behandlungen ausgewählt werden aus thermischen, mechanischen und chemischen Prozessen oder Kombinationen davon.

4. Verfahren gemäß Anspruch 3, wobei die Ernte-Fermentationslösung vor der Ultrafiltration auf einen pH-Wert von 3,5 bis 1,5 angesäuert wird.

5. Verfahren gemäß Anspruch 4, wobei die Ansäuerung auf einen pH-Wert von 3,5 bis 2,0 mit einer Erwärmung auf Temperaturen zwischen 50 und 70°C kombiniert wird.

6. Verfahren gemäß Anspruch 4 oder 5, wobei eine starke Säure auch mit ihrer maximal möglicher Konzentration verwendet wird, zum Beispiel ausgewählt aus Schwefelsäure, Salzsäure, Phosphorsäure oder Trifluoressigsäure.

7. Verfahren gemäß einem der Ansprüche 1 bis 6, wobei spiralige Membranen mit einem Molekulargewicht-Cut-Off von 5.000 bis 300.000 Dalton verwendet werden.

8. Verfahren gemäß einem der Ansprüche 1 bis 7, wobei auf die Ultrafiltration eine komplette Hydrolyse des Polysaccharids mittels Behandlung mit einer starken Säure folgt.

9. Verfahren gemäß Anspruch 8, wobei auf die Hydrolyse eine Neutralisation mittels einer Base folgt.

10. Verfahren gemäß Anspruch 9, wobei die neutralisierte Mischung einer Behandlung zur Entfernung von Glukose, Galaktose oder anderen Zuckern, die das Polysaccharid bilden, unterzogen wird.

11. Verfahren gemäß Anspruch 10, wobei nach optionaler Filtration oder Ultrafiltration die entstandene Mischung mittels lonenaustauscherharzen deionisiert wird und schließlich die L-Fukose durch Zugabe eines Lösungsmittels, ausgewählt aus Methanol, Ethanol, n-Propanol, Isopropanol oder 2-Butanon, oder direkt in Wasser kristallisiert wird.

12. Verfahren zum Abtrennen eines extrazellulären Polysaccharids, das L-Fukose aufweist und in einer Ernte-Fermentationslösung eines Mikroorganismus vorhanden ist, wobei das Verfahren **dadurch gekennzeichnet ist, dass** die Ernte-Fermentationslösung für einen ersten Reinigungsschritt einer Ultrafiltration unterzogen wird, um im Retentat das Polysaccharid zusammen mit den Zellen abzutrennen und um im Permeat quantitativ Salze und Rückstände der verschiedenen Kohlenstoffquellen, die bei der Fermentation benutzt wurden, sowie die Nebenprodukte der Fermentation im Permeat zu entfernen;
wobei die Ultrafiltration durchgeführt wird mit der Ernte-Fermentationslösung, wie sie am Ende der Ernte-Fermentation vorliegt, oder nachdem die Lösung, wie sie am Ende der Fermentation vorliegt, Fluidisierungs-Behandlungen unterzogen wurde;
wobei das Polysaccharid in der Ernte-Fermentationslösung des Mikroorganismus DMS22227 ausgenommen ist.

## Revendications

1. Procédé pour isoler du L-fucose d'un polysaccharide, ledit polysaccharide comprenant du L-fucose, ledit polysaccharide étant contenu dans un bouillon de fermentation de récolte d'un micro-organisme, ledit procédé étant **caractérisé en ce que** le bouillon de fermentation de récolte est soumis, en tant que premier traitement de purification, à une ultrafiltration pour isoler le polysaccharide conjointement avec les cellules dans le rétentat, et pour éliminer quantitativement dans le perméat des sels et résidus des diverses sources de carbone, qui peuvent avoir été utilisées pour la fermentation conjointement avec les sous-produits résultant de la fermentation ;
dans lequel l'ultrafiltration est réalisée sur le bouillon de fermentation de récolte tel qu'il est obtenu à la fin de la fermentation de la récolte ou après avoir soumis le bouillon tel qu'il est obtenu à la fin de la fermentation à des traitements de fluidisation ;
en excluant le polysaccharide contenu dans le bouillon de fermentation de récolte du micro-organisme DMS22227.

2. Procédé selon la revendication 1, dans lequel le micro-organisme appartient au genre *Enterobacter* ou *Clavibacter.*

3. Procédé selon la revendication 2, dans lequel les traitements de fluidisation sont choisis parmi les procédés thermiques, mécaniques et chimiques ou une combinaison de ceux-ci.

4. Procédé selon la revendication 3, dans lequel le bouillon de fermentation de récolte est acidifié à un pH de 3,5 à 1,5 avant d'être ultrafiltré.

5. Procédé selon la revendication 4, dans lequel l'acidification à un pH de 3,5 à 2,0 est combinée à un chauffage à des températures de 50 à 70 °C.

6. Procédé selon la revendication 4 ou 5, dans lequel un acide fort est également utilisé à sa concentration possible maximale, par exemple choisi parmi l'acide sulfurique, l'acide chlorhydrique, l'acide phosphorique, l'acide trifluoroacétique.

7. Procédé selon l'une quelconque des revendications 1 à 6, dans lequel des membranes en spirale sont utilisées, avec un seuil de coupure en masse moléculaire de 5000 à 300 000 Daltons.

8. Procédé selon l'une quelconque des revendications 1 à 7, dans lequel l'ultrafiltration est suivie d'une hydrolyse complète du polysaccharide au moyen d'un traitement avec un acide fort.

9. Procédé selon la revendication 8, dans lequel l'hydrolyse est suivie d'une neutralisation à l'aide d'une base.

10. Procédé selon la revendication 9, dans lequel le mélange neutralisé est soumis à un traitement pour éliminer le glucose, le galactose ou d'autres sucres qui forment le polysaccharide.

11. Procédé selon la revendication 10, dans lequel, après une filtration ou une ultrafiltration facultative, le mélange résultant est désionisé en passant sur des résines échangeuses d'ions, et enfin le L-fucose est cristallisé en ajoutant un solvant choisi parmi le méthanol, l'éthanol, le n-propanol, l'isopropanol ou la 2-butanone, ou directement dans de l'eau.

12. Procédé pour isoler un polysaccharide extracellulaire comprenant du L-fucose et contenu dans un bouillon de fermentation de récolte d'un micro-organisme, ledit procédé étant **caractérisé en ce que** le bouillon de fermentation de récolte est soumis, en tant que première étape de purification, à une ultrafiltration pour isoler le polysaccharide conjointement avec les cellules dans le rétentat, et pour éliminer quantitativement dans le perméat des sels et résidus des diverses sources de carbone, qui peuvent avoir été utilisées pour la fermentation conjointement avec les sous-produits résultant de la fermentation ; dans lequel l'ultrafiltration est réalisée sur le bouillon de fermentation de récolte tel qu'il est obtenu à la fin de la fermentation de récolte ou après avoir soumis le bouillon tel qu'il est obtenu à la fin de la fermentation à des traitements de fluidisation ;
en excluant le polysaccharide contenu dans le bouillon de fermentation de récolte du micro-organisme DMS22227.
